# FASCICULE DE BREVET EUROPEEN

(11) **EP 2 676 657 B1**
(45) Date de publication et mention de la délivrance du brevet: **28.03.2018**
(21) Numéro de dépôt: 12174817.2
(22) Date de dépôt: 03.07.2012
(51) Int. Cl.: A61K 31/194, A61K 33/10, A61K 9/46, A61K 9/20, A61K 9/00

(54) **COMPOSITION POUR LE TRAITEMENT DE L'HYPOCALCÉMIE CHEZ LES RUMINANTS**
ZUSAMMENSETZUNG FÜR DIE BEHANDLUNG VON HYPOKALZÄMIE BEI WIEDERKÄUERN
COMPOSITION FOR THE TREATMENT OF HYPOCALCAEMIA IN RUMINANTS

(30) Priorité: 22.06.2012 FR 1255941
(43) Date de publication de la demande: 25.12.2013
(73) Titulaire: HY-NUTRITION, 35800 Dinard (FR)
(72) Inventeur: Le Jean, Gilles, 35350 Coulomb (FR)
(74) Mandataire: Chantraine, Sylvie Hélène

(56) Documents cités:
- US-A- 5 601 836
- R. P. HANZLIK ET AL: "Relative Bioavailability of Calcium from Calcium Formate, Calcium Citrate, and Calcium Carbonate", JOURNAL OF PHARMACOLOGY AND EXPERIMENTAL THERAPEUTICS, vol. 313, no. 3, 1 juin 2005 (2005-06-01), pages 1217-1222, XP055043055, ISSN: 0022-3565, DOI: 10.1124/jpet.104.081893

## Description

L'invention a pour objet un produit vétérinaire ou un produit de nutrition destiné en particulier à la prévention ou au traitement des hypocalcémies chez les animaux ruminants.

Chez les vaches hautes productrices, le début de lactation pose un problème majeur en matière de métabolisme du calcium. La montée rapide de la lactation dès le vêlage augmente rapidement les besoins en calcium. La vache, qui sort de gestation, n'est pas habituée à déstocker ses réserves de calcium corporelles, et il faut quelques jours pour que les mécanismes de ce déstockage se mettent en place. Il s'en suit donc une période critique de quelques jours, juste après le vêlage, pendant laquelle la vache fraîchement vêlée peine à maintenir sa calcémie à un niveau satisfaisant, c'est-à-dire de l'ordre de 80 mg/l. Lorsque la calcémie descend en dessous de 80 mg/l, la vache entre en hypocalcémie sub-clinique, peu symptomatique, mais dont on sait qu'elle altère les performances de production laitière. Lorsque la calcémie descend en dessous de 50 mg/l, la vache ne tient plus sur ses aplombs et se couche. Si rien n'est fait, le pronostic vital est engagé. La vache est alors au stade clinique de la fièvre vitulaire encore appelée « fièvre de lait » ou « hypocalcémie post partum ».

La fièvre vitulaire clinique est généralement traitée par injection intraveineuse de calcium afin de restaurer la calcémie. Cette intervention curative est du ressort de la médecine vétérinaire.

Pour limiter la fréquence de ces cas extrêmes et graves, il est possible de réaliser une prévention nutritionnelle du risque de fièvre vitulaire en traitant les vaches autour du vêlage avant qu'elles ne soient en fièvre vitulaire sub-clinique.

Les premiers compléments nutritionnels utilisés dans cette indication ont été des gels de sels minéraux ou organiques de calcium.

Un gel à base de propionate de calcium et d'oxyde de calcium a ainsi été décrit dans le brevet US 5 601 836.

A titre d'autre exemple, le produit CALFORM® de Bayer Animal Health est un gel oral commercialisé en flacons de 350 ml et contenant 165 g de formiate de calcium, soit 50 g de calcium, le formiate de calcium étant reconnu comme une forme moins irritante que le chlorure de calcium, et de bonne biodisponibilité. La posologie s'établit comme suit : 350 ml la veille du vêlage, 350 ml au vêlage, 350 ml 12 heures après vêlage et 350 ml 24 heures après vêlage. Cette technique est efficace mais présente deux inconvénients. Le produit est administré en déglutition lente et
l'animal doit avoir la tête relevée, ce qui nécessite une contention de l'animal pendant tout le temps de la déglutition. Dans les faits, l'éleveur ne respecte pas toujours ces consignes de distribution et il s'en suit de fausses déglutitions, c'est-à-dire le passage du produit dans la trachée artère avec des conséquences pulmonaires graves et douloureuses pour l'animal.

Pour pallier ces inconvénients, des produits ont été développés sous forme de bolus, c'est-à-dire de grosses tablettes orales. Ces bolus ont pour avantage d'être d'administration beaucoup plus rapide (de l'ordre d'une minute) et sans risque de fausse déglutition. Un produit de ce type est commercialisé sous la marque BOVIKALC® par Boehringer Ingelheim.

Le BOVIKALC® pèse 190 g et contient 42 g de calcium sous forme de chlorure de calcium (67% de la formule) et de sulfate de calcium (27% de la formule). La posologie suit un rythme proche de celle des gels, c'est-à-dire au minimum un bolus avant vêlage et un bolus 12 heures plus tard.

La forme galénique de BOVIKALC® est incontestablement un progrès par rapport au produit CALFORM® mais la composition du produit n'est pas satisfaisante. BOVIKALK® apporte du calcium sous deux formes :
- du chlorure de calcium qui est certes une source de calcium rapidement métabolisé mais qui est agressif pour les muqueuses et notamment la muqueuse oesophagienne. C'est pourquoi le BOVIKALC est recouvert d'une fine enveloppe protectrice et que la notice stipule que celle-ci doit rester intacte jusqu'à l'administration à l'animal.
- du sulfate de calcium, bien connu comme constituant du plâtre, et qui a plus un rôle de plastifiant du bolus qu'un rôle nutritionnel.

Enfin, des bolus effervescents ont déjà été proposés mais ils ne sont pas administrés à sec. Ils sont au contraire dilués dans l'eau de boisson des animaux.

L'appareil digestif des animaux ruminants présente la particularité de comporter un énorme réservoir appelé réticulorumen, stratifié en trois phases de consistance différente : une phase gazeuse au-dessus d'une phase solide qui flotte elle-même au-dessus d'une phase liquide. Seule la phase liquide, chargée de microparticules passe dans la suite du tractus digestif.

Un aliment ou un principe actif solide séjourne plus longtemps dans le réticulorumen qu'un liquide et son absorption dans le système sanguin est de fait moins rapide. A contrario, un principe actif distribué sous forme liquide, ou sous forme hydrodispersible, entre plus rapidement en contact avec le système sanguin de l'animal soit par le biais d'une absorption par la paroi ruminale, soit par le biais d'un passage plus rapide à des fins d'absorption dans les parties suivantes du tube digestif.

En ce qui concerne l'administration forcée de principes actifs aux animaux ruminants, et pour des raisons de praticité, l'utilisation des formes dites « bolus » est avantageuse. Le bolus est un dispositif solide placé par l'opérateur au-delà du bourrelet lingual de l'animal que ce dernier déglutit, le plaçant de lui-même dans le réticulorumen.

Compte tenu des particularités du réticulorumen décrites ci-dessus, la forme bolus ne permet pas une absorption aussi rapide des principes actifs que les formes liquides. Toutefois, il est plus facile de faire ingérer un bolus qu'un liquide, à un animal ruminant. Or, dans certaines maladies ou dans certains troubles, notamment la fièvre vitulaire, la rapidité d'absorption du principe actif et la constance de sa concentration sanguine au cours du temps sont déterminantes pour garantir l'efficacité du traitement.

Des études ont été réalisées chez l'homme pour comparer la biodisponibilité de différents sels de calcium. Par exemple Hanzlik et al. ont montré que le formiate de calcium est doté d'une meilleure biodisponibilité que le citrate de calcium et le carbonate de calcium chez l'homme (J. Pharm. Exp. Ther., 2005, pp 1217-1222). Ces résultats ne sont cependant pas extrapolables aux ruminants.

Compte tenu de l'art antérieur, il subsiste donc encore le besoin de bénéficier de la facilité d'administration du bolus tout en apportant du calcium avec des sels non agressifs et biodisponibles. Par ailleurs, il est important de séquencer les apports de calcium pour éviter une calcémie en dent de scie, sans pour autant multiplier les gestes d'administration du soigneur.

Il a été découvert dans le cadre de la présente invention qu'un bolus comprenant la combinaison de carbonate de calcium et de formiate de calcium permet d'augmenter la biodisponibilité du calcium et d'accélérer son absorption par rapport aux produits bolus existants pour la même indication. Un bolus au sens de l'invention est un comprimé de poudres destiné à être administré à un animal.

Le bolus a avantageusement une densité et un poids tels qu'il tombe directement dans la phase liquide du réticulum.

Le carbonate de calcium représente de préférence de 5 à 50 % en poids du poids du bolus. Le formiate de calcium représente de préférence de 5 à 60% en poids du poids du bolus. Selon un mode de mise en oeuvre, le carbonate de calcium représente de 35 à 40% en poids du poids du bolus, et le formiate de calcium représente de 5 à 15% en poids du poids du bolus. Selon un autre mode de mise en oeuvre, le carbonate de calcium représente de 5 à 15% en poids du poids du bolus, et le formiate de calcium représente de 50 à 55% en poids du poids du bolus.

Le bolus, lorsqu'il est préparé par compression de poudres, comprend avantageusement tout additif nécessaire à sa mise en forme, notamment des agents de charges et des agents lubrifiants nécessaires à la compression des poudres pour obtenir une forme solide administrable sous la forme d'un gros comprimé. Les agents de charge sont généralement choisis parmi les sucres, tels que le lactose ou le sorbitol. Les agents lubrifiants sont par exemple le stéarate de calcium ou le stéarate de magnésium.

L'agent lubrifiant est avantageusement le stéarate de calcium.

Le bolus n'est avantageusement pas enveloppé dans un film protecteur au moment de son administration ; son poids est généralement compris entre 50 et 200 g.

Le bolus contient de préférence entre 10 g et 50 g de calcium, de préférence encore entre 15 et 25 g de calcium, sous la forme de carbonate de calcium et de formiate de calcium. Le poids du carbonate de calcium et du formiate de calcium représente donc de préférence entre 40 et 70% en poids du poids du bolus.

L'invention a également pour objet le bolus qui vient d'être décrit pour son utilisation dans le traitement ou la prévention de l'hypocalcémie chez des animaux ruminants.

L'hypocalcémie au sens de l'invention n'est pas nécessairement un trouble métabolique. Sa prévention ne relève pas systématiquement de la prévention d'une maladie. L'hypocalcémie au sens de l'invention est une calcémie inférieure ou égale à 80 mg/l.

Les animaux ruminants sont par exemple des bovins, des ovins, des caprins ou des buffles, de préférence encore des femelles présentant des besoins accrus en calcium, notamment des femelles en lactation, par exemple des vaches laitières.

Le bolus de l'invention trouve un intérêt tout particulier dans le traitement de prévention des hypocalcémies chez les femelles bovines en début de lactation, et en particulier pour la prévention de la fièvre vitulaire.

La fièvre vitulaire au sens de l'invention est un trouble métabolique situé autour du part de la vache laitière.

Le bolus est de préférence administré dès les signes précurseurs du vêlage ou lors du vêlage de la vache. Cet apport restaure le niveau de calcium dans le sang et le maintient au-dessus du seuil critique, de préférence au-dessus de 80 mg/l, réduisant ainsi le risque de fièvre vitulaire. Le bolus est de préférence administré avant tout signe clinique d'hypocalcémie ou avant toute chute de la calcémie, pour éviter tout traitement complémentaire ultérieur et notamment des perfusions de gluconate de calcium.

Dans un mode de mise en oeuvre, un deuxième bolus est administré au moins 12 heures après le premier. Le bolus peut favoriser le maintien d'un bon état général pour soutenir la production laitière ou augmenter la production laitière.

Selon un mode de réalisation, le bolus comprend en outre un acide minéral ou organique pour que le bolus entre en effervescence une fois qu'il est en contact avec le liquide reticulo-ruminal après son administration.

On utilise de préférence un acide organique, et de préférence encore l'acide citrique anhydre, car l'acide citrique réagit avec le carbonate de calcium pour former du citrate de calcium, qui est plus absorbable dans l'intestin que le carbonate de calcium.

On préfère que les quantités d'acide organique et de carbonate de calcium soient telles que la réaction d'effervescence se poursuive pendant au moins 10 minutes, de préférence au moins 15 minutes, de préférence encore au moins 20 minutes dans le rumen de l'animal.

Dans ce mode de réalisation, la libération de calcium rapide sous forme d'un sel de formiate est combinée à la libération d'un sel de calcium très absorbable, ce qui a pour effet la remontée rapide et significative du taux de calcium sanguin.

L'effervescence libère du formiate de calcium soluble qui s'hydrolyse en Ca²⁺ absorbé soit par la paroi ruminale soit dans l'intestin. L'effervescence transforme le carbonate de calcium en citrate de calcium lequel est très peu soluble mais plus absorbable dans l'intestin que le carbonate.

Le bolus effervescent permet de réduire le risque de fièvre de lait, grâce à une absorption rapide et élevée du calcium sous forme de formiate et de citrate.

Selon un mode de réalisation, le bolus n'est pas effervescent.

Dans un mode de réalisation avantageux, l'invention a pour objet un ensemble d'un premier bolus et d'un deuxième bolus, lesquels comprennent chacun au moins un sel de calcium, le premier bolus libérant du calcium dans l'organisme d'un animal ruminant à une vitesse supérieure à celle du deuxième bolus. Les deux bolus présentent avantageusement des cinétiques de libération du calcium différentes, si bien que le premier par exemple libère du calcium de façon rapide et le deuxième libère du calcium de façon différée dans le temps, pour prolonger l'effet du premier, et maintenir une calcémie stable.

Le premier bolus est de préférence anhydre, au sens où il contient moins de 1 % en poids d'eau. Le sel de calcium est de préférence choisi parmi les sels organiques et inorganiques, notamment les sels de gluconate, formiate, citrate, carbonate et stéarate de calcium.

Les deux bolus sont de préférence conformes au bolus qui a été décrit précédemment.

Selon un mode de réalisation, le premier bolus peut être par exemple un bolus effervescent et le deuxième bolus n'entre pas de préférence en effervescence lorsqu'il atteint le rumen, car il est destiné à une libération plus lente du calcium.

Le premier bolus contient de préférence du formiate de calcium et un couple effervescent composé de préférence d'acide citrique anhydre et de carbonate de calcium. Le premier bolus tombe dans le réseau où il commence le processus d'effervescence au contact du jus réticulo-ruminal. L'acide citrique se combine avec le carbonate de calcium pour former du citrate de calcium, connu pour sa biodisponibilité. Dans le même temps, le phénomène d'effervescence accélère la diffusion du formiate de calcium dans le jus réticulo-ruminal et donc son accès aux sites d'absorption. Le premier bolus se dissout in vitro à 40°C dans du jus de rumen en une durée comprise entre 5 minutes et 1 heure, par exemple entre 10 et 40 minutes.

Le deuxième bolus contient de préférence du formiate de calcium et du carbonate de calcium mais il n'est pas de préférence effervescent. Le deuxième bolus, une fois administré à l'animal, tombe dans le réseau où il commence le processus de délitement par simple contact avec le jus réticulo-ruminal. Le deuxième bolus se dissout in vitro à 40°C dans du jus de rumen en une durée comprise entre 1 et 10 heures, par exemple entre 6 et 8 heures.

Selon un mode de réalisation de l'invention :
- dans le premier bolus, le carbonate de calcium représente de 35 à 40% en poids du bolus, et le formiate de calcium représente de 5 à 15% en poids du poids du bolus, et
- dans le deuxième bolus, le carbonate de calcium représente de 5 à 15% en poids du bolus, et le formiate de calcium représente de 50 à 55% en poids du poids du bolus.

L'association des deux bolus peut apporter en une seule administration du carbonate de calcium (qui se transforme partiellement en citrate de calcium), et du formiate de calcium, le tout avec des rythmes de libération différents, permettant ainsi d'étaler l'absorption du calcium entre deux prises, et éviter des variations trop brusques de calcémie chez l'animal.

Les administrations du premier et du deuxième bolus sont avantageusement faites simultanément grâce à un applicateur permettant d'administrer les deux bolus en un seul geste ; à défaut elles sont successives et ne sont pas espacées dans le temps de plus de 15 minutes, de préférence de plus de 5 minutes.

On administre de préférence au moins deux ensembles de bolus tels que décrits précédemment. Un premier ensemble de bolus est de préférence administré au ruminant femelle dès les premiers signes du vêlage ou juste après le vêlage. Un deuxième ensemble de bolus est avantageusement administré au moins 12 heures après le premier.

Cet apport de calcium, en deux bolus à vitesse de délitement différente, et trois sels de calcium différents est une innovation de principe sur le marché.

Cette innovation est efficace comme produit de nutrition, notamment pour la prévention de la fièvre vitulaire chez les vaches laitières.

L'invention est illustrée plus en détail par les exemples suivants.

### Exemple 1 : Bolus de calcium selon l'invention à libération rapide

On a préparé un bolus effervescent de 105 g ayant la composition suivante. Les pourcentages sont en poids.

| | |
|---|---|
| carbonate de calcium | 37,7% |
| acide citrique | 15,0% |
| sorbitol | 36% |
| formiate de calcium | 10% |
| Stéarate de calcium | 1,3% |

Les essais in vitro dans du jus de rumen à 40°C environ ont montré une dissolution complète du bolus en 30 minutes.

### Exemple 2 : Bolus de calcium selon l'invention à libération lente

On a préparé un bolus effervescent de 105 g ayant la composition suivante. Les pourcentages sont en poids.

| | |
|---|---|
| carbonate de calcium | 10 % |
| sorbitol | 33,9 % |
| formiate de calcium | 52,7 % |
| PEG 6000 | 2,4 % |
| stéarate de calcium | 1% |

Les essais in vitro dans du jus de rumen à 40°C environ ont montré une dissolution complète du bolus en 7 heures.

### Exemple 3 : Etude sur animaux

Une expérimentation a été menée par un docteur vétérinaire de terrain, en situation d'élevage normale, l'objectif étant de mesurer l'évolution de la calcémieau cours du temps chez quatre vaches et d'évaluer le degré de prévention de la fièvre vitulaire.

Les vaches choisies présentaient toutes des risques ou des antécédents de fièvre vitulaire. On leur a administré de façon concomitante les bolus des exemples 1 et 2, à une ou plusieurs reprises, et leur calcémie a été mesurée au cours du temps.

### Vache 1 : Race PrimHolstein de 5 ans, en troisième lactation, sortant d'une grosse lactation (12932 Kg) et trop grasse au vêlage.

| | Administration simultanée des deux bolus | Calcémie (mg/l) | Verbatim du commentaire du vétérinaire |
|---|---|---|---|
| Avant Vêlage | Oui | Nd | Situation à risque. Bons résultats. La calcémie se tient. Pas de problèmes ultérieurs. |
| Vêlage | Oui | 80 | |
| Vêlage +3 h | | 82 | |
| Vêlage +6 h | | 80 | |
| Vêlage + 12 h | Oui | 77 | |
| Vêlage + 24 h | | 72 | |

### Vache 2 : Race PrimHolstein de 4 ans, en troisième lactation, sortant d'une grosse lactation (9265 Kg).

| | Administration simultanée des deux bolus | Calcémie (mg/l) | Verbatim du commentaire du vétérinaire |
|---|---|---|---|
| Vêlage -2 h | Oui | 81 | Situation à risque. Bons résultats. La calcémie se tient. Le traitement est efficace. Aucun problème ultérieur |
| Vêlage | Oui | 77 | |
| Vêlage +3 h | | 82 | |
| Vêlage +5 h | | 95 | |
| Vêlage + 11 h | Oui | 84 | |
| Vêlage + 24 h | | 75 | |

### Vache 3 : Race PrimHolstein de 4 ans, en troisième lactation, sortant d'une lactation normale (7903 Kg).

| | Administration simultanée des deux bolus | Calcémie (mg/l) | Verbatim du commentaire du vétérinaire |
|---|---|---|---|
| Vêlage -3 h | Oui | 84 | Situation à risque modéré. Bons résultats. La calcémie se tient. Le traitement est efficace. Aucun problème ultérieur |
| Vêlage | Oui | 86 | |
| Vêlage +3 h | | 85 | |
| Vêlage +5 h | | 84 | |
| Vêlage + 11 h | Oui | 86 | |
| Vêlage + 24 h | | 81 | |

### Vache 4 : Race PrimHolstein de 6 ans, en quatrième lactation, sortant d'une lactation normale (8555 kg).

| | Administration simultanée des deux bolus | Calcémie (mg/l) | Verbatim du commentaire du vétérinaire |
|---|---|---|---|
| Avant vêlage | Non | Nd | Situation à risque moyens. Le traitement est efficace. Aucun problème ultérieur |
| Vêlage | Oui | 78 | |
| Vêlage +3 h | | Nd | |
| Vêlage +6 h | | 81 | |
| Vêlage + 12 h | Oui | 75 | |
| Vêlage + 26 h | | 77 | |

Dans les quatre cas ci-dessus, la calcémie est restée quasi linéaire aux environs de 80 mg/litre. Les résultats démontrent l'efficacité zootechnique du bolus de l'invention sur la prévention de la fièvre vitulaire. Cette efficacité est renforcée lorsque l'administration du bolus est réalisée avant que l'hypocalcémie ne soit trop déclarée.

## Revendications

1. Bolus comprenant du carbonate de calcium et du formiate de calcium, dans lequel le carbonate de calcium représente de 5 à 50 % en poids du bolus, et le formiate de calcium représente de 5 à 60 % en poids du bolus.

2. Bolus selon la revendication 1, **caractérisé en ce qu'**il comprend en outre au moins un agent de charge et au moins un agent lubrifiant.

3. Bolus selon l'une des revendications 1 à 2, **caractérisé en ce que** l'agent lubrifiant est le stéarate de calcium ou le stéarate de magnésium.

4. Bolus selon la revendication 1, **caractérisé en ce qu'**il comprend en outre un acide minéral ou organique.

5. Bolus selon l'une des revendications 1 à 4 pour son utilisation dans le traitement ou la prévention de l'hypocalcémie chez des animaux ruminants.

6. Bolus selon la revendication 1, **caractérisé en ce que** le carbonate de calcium représente de 35 à 40% en poids du poids du bolus, et le formiate de calcium représente de 5 à 15% en poids du poids du bolus.

7. Bolus selon la revendication 1, **caractérisé en ce que** le carbonate de calcium représente de 5 à 15% en poids du poids du bolus, et le formiate de calcium représente de 50 à 55% en poids du poids du bolus.

8. Ensemble d'un premier bolus et d'un deuxième bolus **caractérisé en ce que**, dans le premier bolus, le carbonate de calcium représente de 35 à 40% en poids du poids du bolus, et le formiate de calcium représente de 5 à 15% en poids du poids du bolus, et **en ce que**, dans le deuxième bolus, le carbonate de calcium représente de 5 à 15% en poids du poids du bolus, et le formiate de calcium représente de 50 à 55% en poids du poids du bolus.

## Patentansprüche

1. Bolus, umfassend Calciumcarbonat und Calciumformiat, wobei Calciumcarbonat 5 bis 50 Gew.-% des Bolus darstellt und Calciumformiat 5 bis 60 Gew.-% des Bolus darstellt.

2. Bolus gemäß Anspruch 1, **dadurch gekennzeichnet, dass** er ferner mindestens einen Füllstoff und mindestens ein Schmiermittel enthält.

3. Bolus gemäß einem der Ansprüche 1 bis 2, **dadurch gekennzeichnet, dass** es sich bei dem Schmiermittel um Calciumstearat oder Magnesiumstearat handelt.

4. Bolus gemäß Anspruch 1, **dadurch gekennzeichnet, dass** er ferner eine mineralische oder organische Säure enthält.

5. Bolus gemäß einem der Ansprüche 1 bis 4 zur Verwendung bei der Behandlung oder Vorbeugung von Hypokalzämie bei Wiederkäuern.

6. Bolus gemäß Anspruch 1, **dadurch gekennzeichnet, dass** Calciumcarbonat 35 bis 40 Gew.-% des Gewichts des Bolus darstellt und Calciumformiat 5 bis 15 Gew.-% des Gewichts des Bolus darstellt.

7. Bolus gemäß Anspruch 1, **dadurch gekennzeichnet, dass** Calciumcarbonat 5 bis 15 Gew.-% des Gewichts des Bolus darstellt und Calciumformiat 50 bis 55 Gew.-% des Gewichts des Bolus darstellt.

8. Set aus einem ersten Bolus und einem zweiten Bolus, **dadurch gekennzeichnet, dass** in dem ersten Bolus Calciumcarbonat 35 bis 40 Gew.-% des Gewichts des Bolus darstellt und Calciumformiat 5 bis 15 Gew.-% des Gewichts des Bolus darstellt, und dass in dem zweiten Bolus Calciumcarbonat 5 bis 15 Gew.-% des Gewichts des Bolus darstellt und Calciumformiat 50 bis 55 Gew.-% des Gewichts des Bolus darstellt.

## Claims

1. Bolus comprising calcium carbonate and calcium formate, wherein calcium carbonate represents from 5% to 50% by weight of the weight of the bolus, and calcium formate represents from 5% to 60% of the bolus.

2. Bolus comprising calcium carbonate and calcium formate, wherein it further comprises at least one filler and at least one lubricant.

3. Bolus comprising calcium carbonate and calcium formate, wherein the lubricant is calcium stearate or magnesium stearate.

4. Bolus comprising calcium carbonate and calcium formate, wherein it further comprises a mineral or organic acid.

5. for use in the treatment or prevention of hypocalcaemia in the ruminant animal in need thereof.

6. wherein calcium carbonate represents from 35% to 40% by weight of the weight of the bolus, and calcium formate represents from 5% to 15% of the bolus.

7. wherein calcium carbonate represents from 5% to 15% by weight of the weight of the bolus, and calcium formate represents from 50% to 55% of the bolus.

8. Set of a first bolus and a second bolus, wherein, in the first bolus, calcium carbonate represents from 35% to 40% by weight of the weight of the bolus, and calcium formate represents from 5% to 15% of the bolus, and wherein, in the second bolus, calcium carbonate represents from 5% to 15% by weight of the weight of the bolus, and calcium formate represents from 50% to 55% by weight of the weight of the bolus.
